(19) **Europäisches Patentamt**
**European Patent Office**
**Office européen des brevets**

(11) **EP 3 311 752 B1**

(12) **EUROPEAN PATENT SPECIFICATION**

(45) Date of publication and mention
of the grant of the patent:
**25.11.2020 Bulletin 2020/48**

(21) Application number: **16811860.2**

(22) Date of filing: **09.06.2016**

(51) Int Cl.:
*G16H 50/20* (2018.01)    *A61B 8/00* (2006.01)
*A61B 8/08* (2006.01)    *G01S 7/52* (2006.01)
*G01S 15/89* (2006.01)

(86) International application number:
**PCT/KR2016/006105**

(87) International publication number:
**WO 2016/204447 (22.12.2016 Gazette 2016/51)**

(54) **ULTRASONIC DEVICE AND OPERATION METHOD THEREFOR**

ULTRASCHALLVORRICHTUNG UND BETRIEBSVERFAHREN DAFÜR

DISPOSITIF À ULTRASONS ET SON PROCÉDÉ DE FONCTIONNEMENT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.06.2015 US 201562180145 P
13.01.2016 KR 20160004413**

(43) Date of publication of application:
**25.04.2018 Bulletin 2018/17**

(73) Proprietor: **Samsung Medison Co., Ltd.
Hongcheon-gun, Gangwon-do 25108 (KR)**

(72) Inventors:
• **DAFT, Christopher M.W
Hongcheon-gun
Gangwon-do 25108 (KR)**
• **KIM, Hyoung-jin
Hongcheon-gun
Gangwon-do 25108 (KR)**
• **KIM, Kang-sik
Seongnam-si
Gyeonggi-do 13517 (KR)**
• **LEE, Woo-youl
Hongcheon-gun
Gangwon-do 25108 (KR)**

(74) Representative: **Hylarides, Paul Jacques et al
Arnold & Siedsma
Bezuidenhoutseweg 57
2594 AC Den Haag (NL)**

(56) References cited:
JP-A- 2007 117 168    JP-A- 2007 296 336
JP-A- 2013 244 164    JP-A- 2014 068 755
KR-A- 20040 070 404    US-A- 5 873 830
US-A1- 2010 305 441

• **KIM KYUHONG ET AL: "A fast minimum variance
beamforming method using principal component
analysis", IEEE TRANSACTIONS ON
ULTRASONICS, FERROELECTRICS AND
FREQUENCY CONTROL, IEEE, US, vol. 61, no. 6,
1 June 2014 (2014-06-01), pages 930-945,
XP011548773, ISSN: 0885-3010, DOI:
10.1109/TUFFC.2014.2989 [retrieved on
2014-05-20]**
• **JUNSEOB SHIN ET AL: "Synergistic
enhancements of ultrasound image contrast with
a combination of phase aberration correction and
dual apodization with cross-correlation", IEEE
TRANSACTIONS ON ULTRASONICS,
FERROELECTRICS AND FREQUENCY
CONTROL, IEEE, US, vol. 59, no. 9, 1 September
2012 (2012-09-01), pages 2089-2101,
XP011491495, ISSN: 0885-3010, DOI:
10.1109/TUFFC.2012.2430**

**Description**

TECHNICAL FIELD

The present disclosure relates to ultrasound apparatuses and methods of operating the same, and more particularly, to apparatuses and methods of performing beamforming.

BACKGROUND ART

Ultrasound diagnosis apparatuses transmit ultrasound signals generated by transducers of a probe to an object and receive echo signals reflected from the object, thereby obtaining an image of an object or an internal part of the object. In particular, ultrasound diagnosis apparatuses are used for medical purposes including observing an internal area of an object, detecting foreign substances, and assessing injuries. Such ultrasound diagnosis apparatuses exhibit high stability, display images in real time, and are safe due to there being no radiation exposure, compared to X-ray apparatuses. Therefore, an ultrasound diagnosis apparatus is widely used together with other types of imaging diagnosis apparatuses.

An algorithm applied by such ultrasound diagnosis apparatuses is known from KIM, Kyuhong, et al. A fast minimum variance beamforming method using principal component analysis. IEEE transactions on ultrasonics, ferroelectrics, and frequency control, 2014, 61.6: 930-945. Said article discloses, in its own wording, an MV beamforming method that almost optimally approximates the MV beamforming while reducing the computational complexity greatly through dimensionality reduction using principal component analysis (PCA).

Alternatively, an algorithm applied by such ultrasound diagnosis apparatuses is known from SHIN, Junseob; YEN, Jesse T. Synergistic enhancements of ultrasound image contrast with a combination of phase aberration correction and dual apodization with cross-correlation. IEEE transactions on ultrasonics, ferroelectrics, and frequency control, 2012, 59.9: 2089-2101. Said article discloses, in its own wording, Dual apodization with cross-correlation (DAX) which is an adaptive beamforming technique which utilizes two distinct apodization functions in suppressing side lobes and clutter, which combines DAX with a phase aberration correction algorithm based on nearest-neighbor cross-correlation (NNCC).Furthermore, a method for automatic image optimization in ultrasound imaging of an object is known from US 2010/0305441 A1 which discloses, according to its abstract, a method including transmitting a first ultrasound signal into the object, wherein the signal has a plurality of first signal parameters. The method also includes receiving a first set of electrical signals representing reflections of the first ultrasound signals from the object and processing the first set of electrical signals into a first image. The method evaluates an image quality cost function for the first image to produce a first image quality metric and determines a second plurality of signal parameters based upon the first image quality metric. Similarly, the method includes transmitting a second ultrasound signal into the object, wherein the signal has the second plurality of signal parameters and receiving a second set of electrical signals representing reflections of the second ultrasound signal from the object and processing the second set of electrical signals into a second image. The method also includes evaluating an image quality cost function for the second image to produce a second image quality metric. The method further includes comparing the first image quality metric and the second image quality metric to determine whether a maximized image quality metric has been reached and assigning multiple signal parameters that produced the maximized image quality metric as optimum parameters. The method further includes imaging and displaying the object using an ultrasound signal having the optimum parameters.

Furthermore, an ultrasound system and method for improving resolution and operation is known from US 8,873,830, which discloses, according to its abstract, a system and method which applies different imaging parameters within and outside a region of interest in an ultrasound image to improve spatial and/or temporal resolution inside a region of interest. The system also increases an apparent frame rate within a region of interest in an ultrasound-image frame by generating a motion-compensated interpolated image based on measured motion. The ultrasound imaging system also performs a method for automatically adjusting ultrasound imaging parameters in at least a portion of an ultrasound image in response to transducer or image motion to improve spatial or temporal resolution. With the measured motion, the system can also alter an operating mode of an ultrasound transducer array in response to an absence of transducer motion. Further, the system corrects distortion in an acquired ultrasound image caused by transducer or image motion.

Furthermore, a medical image display device is known from JP 2007 296336 which discloses, according to its abstract, provides a medical image display device of the ultrasonic system which includes a plurality of divided regions and each of the plurality of regions corresponds with one region of the heart, or an imaging object. The medical image display device further includes tracking quality indicators to be displayed related to one or more regions having the tracking qualities lower than a prescribed level in the plurality of divided regions.

Furthermore, an ultrasonic image generation method and ultrasonic image diagnosis equipment is known from JP 2013 244164 which discloses, according to its abstract, an ultrasonic image generation including a second element data acquisition step of acquiring second element data output by transmission based on a second transmission focus condition different from a first transmission focus condition in acquisition of first element data for generating an ultrasonic image; and an optimum sonic velocity calculation step of calculating an optimum sonic velocity value of a region of interest by using the second element data corresponding to the region of interest.

DISCLOSURE

TECHNICAL PROBLEM

Provided are ultrasound imaging apparatuses and methods of operating the same, whereby a more precise ultrasound image may be obtained by detecting a region having low image quality in an ultrasound image and compensating for the low image quality of the region.

Provided is a non-transitory computer-readable recording medium having recorded thereon a program for executing a method of operating an ultrasound imaging apparatus on a computer.

TECHNICAL SOLUTION

According to an aspect of an embodiment, an ultrasound imaging apparatus includes: a probe configured to transmit an ultrasound signal to an object along a first path by focusing the ultrasound signal at a first focal point and receive an echo signal reflected from the object; and a processor configured to generate a first ultrasound image representing the object, detect at least one region having low image quality among regions in the generated first ultrasound image according to a predetermined criterion, wherein the processor is configured to generate a map indicating quality of the first ultrasound image based on the detected at least one region, control the probe based on the map so that the ultrasound signal reaches the detected at least one region along a second path by focusing the ultrasound signal at a second focal point within a predetermined region of the object, wherein the second path is determined based on a factor obstructing the first path and generate a second ultrasound image representing the object based on an echo signal received in response to the ultrasound signal transmitted to the object along the second path. The second path is determined based on a change in at least one of a position of an origin of an ultrasound beam corresponding to the first path and a transmission direction of the ultrasound beam corresponding to the first path.

The probe is further configured to transmit an ultrasound beam composed of the ultrasound signal to the object in a plurality of directions and receive echo signals respectively reflected from the object based on the plurality of directions, and wherein the processor is further configured to detect, according to a predetermined criterion, at least one region having low image quality among regions in the first ultrasound image by using the reflected echo signals.

When detecting the at least one region having low image quality according to the predetermined criterion, the processor detects, if a correlation value for a first focal point, which is acquired using different apodization functions, is less than a predetermined threshold value, a region in the first ultrasound image corresponding to the first focal point as a region having low image quality.

The ultrasound imaging apparatus further includes a display configured to display at least one of the first and second ultrasound images.

The display is further configured to display a map indicating quality of the first ultrasound image based on the detected at least one region.

The display is further configured to display the map in such a manner as to distinguish the at least one region from the other regions excluding the at least one region.

The probe comprises a transducer array consisting of a plurality of transducers, and the plurality of transducers are arranged in a one-dimensional (1D) or two-dimensional (2D) array.

The information about the transmission direction of the ultrasound beam is information about an angle between the transmission direction of the ultrasound beam and the transducer array.

The processor is further configured to control the ultrasound signal to be transmitted along a third path based on the at least one region and generate a second ultrasound image corresponding to the object based on an echo signal received in response to the ultrasound signal transmitted to the object along the second path and an echo signal received in response to the ultrasound signal transmitted to the object along the third path. The ultrasound imaging apparatus further includes a user interface configured to receive a user input for setting transmission of the ultrasound signal along the second path based on the at least one region, and wherein the processor is further configured to control the ultrasound signal to be transmitted along the second path based on the user input.

The processor is further configured to control the probe to perform beamforming by using a predetermined number of sub-apertures into which a plurality of transducers in the probe are divided.

According to an aspect of another embodiment, a method of operating an ultrasound imaging apparatus includes: transmitting, by a probe in the ultrasound imaging apparatus, an ultrasound signal to an object along a first path by focusing the ultrasound signal at a first focal point and receiving an echo signal reflected from the object; generating a first ultrasound image representing the object and detecting at least one region having low image quality among regions in the generated first ultrasound image according to a predetermined criterion; and generating a second ultrasound image representing the object based on an echo signal received in response to the ultrasound signal transmitted to the object along a second path.

Generating the second ultrasound image comprises, generating a map indicating quality of the first ultrasound image based on the detected at least one region; and controlling the probe based on the map so that the ultrasound signal reaches the detected at least one region along the second path by focusing the ultrasound signal at a second focal point within a predetermined region of the object, wherein the second path is determined based a factor obstructing the first path, wherein the second path is determined based on a change in at least one of a position of an origin of an ultrasound beam corresponding to the first path and a transmission direction of the ultrasound beam corresponding to the first path.

The transmitting of the ultrasound signal to the object along the first path and the receiving of the echo signal reflected from the object comprises transmitting an ultrasound beam composed of the ultrasound signal to the object in a plurality of directions and receiving echo signals respectively reflected from the object based on the plurality of directions, and wherein the generating of the first ultrasound image and the detecting of the at least one region having low image quality according to the predetermined criterion comprises detecting, according to a predetermined criterion, at least one region having low image quality among regions in the first ultrasound image by using the reflected echo signals.

The detecting of the at least one region having low image quality according to the predetermined criterion comprises detecting, if a correlation value for a first focal point, which is acquired using different apodization functions, is less than a predetermined threshold value, a region in the first ultrasound image corresponding to the first focal point as a region having low image quality.

The method further includes displaying the second ultrasound images.

The method further includes displaying a map indicating a quality of the first ultrasound image based on the detected at least one region.

The displaying of the map indicating the quality of the first ultrasound image comprises displaying the map in such a manner as to distinguish the at least one region from the other regions excluding the at least one region.

The controlling of the ultrasound signal to be transmitted along the second path comprises controlling the ultrasound signal to be transmitted along a third path based on the at least one region, and wherein the generating of the second ultrasound image representing the object based on the echo signal comprises generating the second ultrasound image representing the object based on an echo signal received in response to the ultrasound signal transmitted to the object along the second path and an echo signal received in response to the ultrasound signal transmitted to the object along the third path.

The method further includes receiving a user input for setting transmission of the ultrasound signal along the second path based on the at least one region, wherein the controlling of the ultrasound signal to be transmitted along the second path comprises controlling the ultrasound signal to be transmitted along the second path based on the user input.

According to an aspect of another embodiment, a non-transitory computer-readable recording medium has recorded thereon a program for executing a method of operating an ultrasound imaging apparatus on a computer, wherein the method includes transmitting an ultrasound signal to an object along a first path and receiving an echo signal reflected from the object; generating a first ultrasound image representing the object and detecting at least one region having low image quality among regions in the generated first ultrasound image according to a predetermined criterion; controlling the ultrasound signal to be transmitted along a second path by focusing the ultrasound signal at a focal point within a predetermined region of the object corresponding to the detected at least one region; and generating a second ultrasound image representing the object based on an echo signal received in response to the ultrasound signal transmitted to the object along the second path.

## ADVANTAGEOUS EFFECTS

An ultrasound imaging apparatus according to an embodiment may obtain a more precise ultrasound image by detecting a region having low image quality in an ultrasound image and compensating for the low image quality of the region.

## DESCRIPTION OF DRAWINGS

Embodiments now will be described more fully hereinafter with reference to the accompanying drawings, in which reference numerals denote structural elements:

FIG. 1 is a block diagram of a configuration of an ultrasound diagnosis apparatus according to an embodiment;

FIG. 2 is a block diagram of a configuration of a wireless probe according to an embodiment;

FIG. 3 is a block diagram of a configuration of an ultrasound imaging apparatus according to an embodiment;

FIG. 4 is a block diagram of a configuration of an ultrasound imaging apparatus according to another embodiment;

FIG. 5 is a flowchart of a method of operating an ultrasound imaging apparatus according to an embodiment;

FIG. 6A is a diagram for explaining detection of a region having low image quality among regions in an ultrasound image, according to an embodiment;

FIG. 6B is a diagram for explaining detection of a region having low image quality among regions in an ultrasound image, according to another embodiment;

FIG. 7A is a diagram for explaining a factor that degrades the quality of an ultrasound image, according to an embodiment;

FIG. 7B is a diagram for explaining a method of improving the quality of an ultrasound image, according to an embodiment;

FIG. 8 is a flowchart of a method of operating an ultrasound imaging apparatus, according to another embodiment;

FIG. 9 is diagram for explaining a first ultrasound image and a map representing a quality of the first ultrasound image, according to an embodiment;

FIG. 10 is a diagram for explaining a first ultrasound image obtained before undergoing improvement of image quality and a second ultrasound image obtained after undergoing improvement of image quality, according to an embodiment;

FIG. 11 is a flowchart of a method of operating an ultrasound imaging apparatus, according to another embodiment; and

FIG. 12 is a diagram for explaining a method of controlling an operation of an ultrasound imaging apparatus based on a user input, according to an embodiment.

BEST MODE

Provided is an ultrasound imaging apparatus including a probe configured to transmit an ultrasound signal to an object along a first path and receive an echo signal reflected from the object; and a processor configured to generate a first ultrasound image representing the object, detect at least one region having low image quality among regions in the generated first ultrasound image according to a predetermined criterion, control the ultrasound signal to be transmitted along a second path by focusing the ultrasound signal at a focal point within a predetermined region of the object corresponding to the detected at least one region, and generate a second ultrasound image representing the object based on an echo signal received in response to the ultrasound signal transmitted to the object along the second path.

MODE FOR INVENTION

The terms used in this specification are those general terms currently widely used in the art in consideration of functions regarding the inventive concept, but the terms may vary according to the intention of those of ordinary skill in the art, precedents, or new technology in the art. Also, some terms may be arbitrarily selected by the applicant, and in this case, the meaning of the selected terms will be described in detail in the detailed description of the present specification. Thus, the terms used herein have to be defined based on the meaning of the terms together with the description throughout the specification.

When a part "includes" or "comprises" an element, unless there is a particular description contrary thereto, the part can further include other elements, not excluding the other elements. Also, the term "unit" in the embodiments of the present invention means a software component or hardware component such as a field-programmable gate array (FPGA) or an application-specific integrated circuit (ASIC), and performs a specific function. However, the term "unit" is not limited to software or hardware. The "unit" may be formed so as to be in an addressable storage medium, or may be formed so as to operate one or more processors. Thus, for example, the term "unit" may refer to components such as software components, object-oriented software components, class components, and task components, and may include processes, functions, attributes, procedures, subroutines, segments of program code, drivers, firmware, micro codes, circuits, data, a database, data structures, tables, arrays, or variables. A function provided by the components and "units" may be associated with the smaller number of components and "units", or may be divided into additional components and "units".

It will be understood that, although the terms "first", "second", etc. may be used herein to describe various elements and/or components, these elements and/or components should not be limited by these terms. These terms are only used to distinguish one element or component from another element or component. For example, a first element or component may be termed a second element or component or vice versa without departing from the teachings of embodiments. As used herein, the term "and/or" includes any and all combinations of one or more of the associated listed items. Expressions such as "at least one of," when preceding a list of elements, modify the entire list of elements and do not modify the individual elements of the list.

Throughout the specification, an "image" may mean multi-dimensional data formed of discrete image elements, e.g., pixels in a two-dimensional (2D) image and voxels in a three-dimensional (3D) image.

Throughout the specification, an "ultrasound image" refers to an image of an object, which is obtained using ultrasound waves. An ultrasound image may be an image obtained by transmitting ultrasound signals generated by transducers of a probe to an object and receiving information about echo signals reflected from the object. Furthermore, an ultrasound image may take different forms. For example, the ultrasound image may be at least one of an amplitude (A) mode image, a brightness (B) mode image, a color (C) mode image, and a Doppler (D) mode image. In addition, according to an embodiment, an ultrasound image may be a 2D or three-dimensional (3D) image.

Furthermore, an "object" may be a human, an animal, or a part of a human or animal. For example, the object may be an organ (e.g., the liver, the heart, the womb, the brain, a breast, or the abdomen), a blood vessel, or a combination thereof. Also, the object may be a phantom. The phantom means a material having a density, an effective atomic number, and a volume that are approximately the same as those of an organism.

Throughout the specification, a "user" may be, but is not limited to, a medical expert, for example, a medical doctor, a nurse, a medical laboratory technologist, or a medical imaging expert, or a technician who repairs medical apparatuses.

Reference will now be made in detail to embodiments, examples of which are illustrated in the accompanying drawings. In this regard, the present embodiments may have different forms and should not be construed as being limited to the descriptions set forth herein.

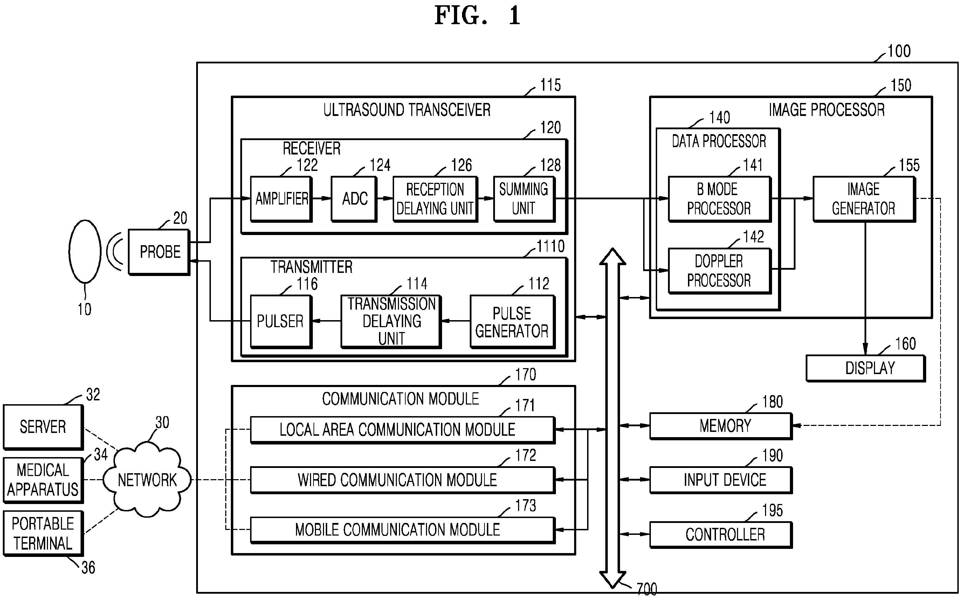

FIG. 1 is a block diagram showing a configuration of an ultrasound diagnosis apparatus 100 according to an embodiment.

Referring to FIG. 1, the ultrasound diagnosis apparatus 100 according to the present embodiment may include a probe 20, an ultrasound transceiver 115, an image processor 150, a display 160, a communication module 170, a memory 180, an input device 190, and a controller 195, which may be connected to one another via a bus 185. The image processor 150 may include an image generator 155, a cross-section information detector 130, and the display 160.

It will be understood by those of ordinary skill in the art that the ultrasound diagnosis apparatus 100 may further include common components other than those shown in FIG. 1.

In some embodiments, the ultrasound diagnosis apparatus 100 may be a cart type apparatus or a portable type apparatus. Examples of portable ultrasound diagnosis apparatuses may include, but are not limited to, a picture archiving and communication system (PACS) viewer, a smartphone, a laptop computer, a personal digital assistant (PDA), and a tablet PC.

The probe 20 transmits ultrasound waves to an object 10 in response to a driving signal applied by the ultrasound transceiver 115 and receives echo signals reflected by the object 10. The probe 20 includes a plurality of transducers, and the plurality of transducers oscillate in response to electric signals and generate acoustic energy, that is, ultrasound waves. Furthermore, the probe 20 may be connected to the main body of the ultrasound diagnosis apparatus 100 by wire or wirelessly, and according to embodiments, the ultrasound diagnosis apparatus 100 may include a plurality of probes 20.

A transmitter 110 supplies a driving signal to the probe 20. The transmitter 110 includes a pulse generator 112, a transmission delaying unit 114, and a pulser 116. The pulse generator 112 generates pulses for forming transmission ultrasound waves based on a predetermined pulse repetition frequency (PRF), and the transmission delaying unit 114 delays the pulses by delay times necessary for determining transmission directionality. The pulses which have been delayed correspond to a plurality of piezoelectric vibrators included in the probe 20, respectively. The pulser 116 applies a driving signal (or a driving pulse) to the probe 20 based on timing corresponding to each of the pulses which have been delayed.

A receiver 120 generates ultrasound data by processing echo signals received from the probe 20. The receiver 120 may include an amplifier 122, an analog-to-digital converter (ADC) 124, a reception delaying unit 126, and a summing unit 128. The amplifier 122 amplifies echo signals in each channel, and the ADC 124 performs analog-to-digital conversion with respect to the amplified echo signals. The reception delaying unit 126 delays digital echo signals output by the ADC 124 by delay times necessary for determining reception directionality, and the summing unit 128 generates ultrasound data by summing the echo signals processed by the reception delaying unit 126.

The image processor 150 generates an ultrasound image by scan-converting ultrasound data generated by the ultrasound transceiver 115.

The ultrasound image may be not only a grayscale ultrasound image obtained by scanning an object in an amplitude (A) mode, a brightness (B) mode, and a motion (M) mode, but also a Doppler image showing a movement of an object. The Doppler image may be a blood flow Doppler image showing flow of blood (also referred to as a color Doppler image), a tissue Doppler image showing a movement of tissue, or a spectral Doppler image showing a moving speed of an object as a waveform.

A B mode processor 141 extracts B mode components from ultrasound data and processes the B mode components. An image generator 155 may generate an ultrasound image indicating signal intensities as brightness based on the extracted B mode components 141.

Similarly, a Doppler processor 142 may extract Doppler components from ultrasound data, and the image generator 155 may generate a Doppler image indicating a movement of an object as colors or waveforms based on the extracted Doppler components.

According to an embodiment, the image generator 155 may generate a 2D or 3D ultrasound image of an object 10 and may also generate an elasticity image by imaging deformation of the object 10 due to pressure. Furthermore, the image generator 155 may display various pieces of additional information in an ultrasound image by using text and graphics. In addition, the generated ultrasound image may be stored in the memory 180.

A display 160 displays the generated ultrasound image. The display 160 may display not only an ultrasound image, but also various pieces of information processed by the ultrasound diagnosis apparatus 100 on a screen image via a graphical user interface (GUI). In addition, the ultrasound diagnosis apparatus 100 may include two or more displays 160 according to embodiments.

The display 160 may include at least one of a liquid crystal display (LCD), a thin film transistor-LCD (TFT-LCD), an organic light-emitting diode (OLED) display, a flexible display, a 3D display, and an electrophoretic display.

Furthermore, when the display 160 and a user input device form a layer structure to form a touch screen, the display 260 may be used not only as an output device but also as an input device via which a user inputs information via a touch.

The touch screen may be configured to detect a position of a touch input, a touched area, and pressure of a touch. The touch screen may also be configured to detect both a real-touch and a proximity-touch.

In the present specification, a 'real-touch' means that a pointer actually touches a screen, and a 'proximity-touch' means that a pointer does not actually touch a screen but approaches the screen while being separated from the screen by a predetermined distance. A 'pointer' used herein means a tool for touching a particular portion on or near a displayed screen. Examples of the pointer may include a stylus pen and a body part such as a finger.

Although not shown, the ultrasound diagnosis apparatus 100 may include various sensors that are disposed within or near the touch screen so as to sense a real-touch or proximity-touch on the touch screen. A tactile sensor is an example of the sensors for sensing a touch on the touch screen.

The tactile sensor is used to sense a touch of a particular object to the same or greater degree than the degree to which a human can sense the touch. The tactile sensor may detect various pieces of information including the roughness of a contact surface, the hardness of an object to be touched, the temperature of a point to be touched, etc.

A proximity sensor is another example of the sensors for sensing a touch. The proximity sensor refers to a sensor that detects the presence of an object that is approaching or is located near a predetermined detection surface by using the force of an electromagnetic field or infrared light without mechanical contact.

Examples of the proximity sensor include a transmissive photoelectric sensor, a direct reflective photoelectric sensor, a mirror reflective photoelectric sensor, a high-frequency oscillation proximity sensor, a capacitive proximity sensor, a magnetic proximity sensor, an infrared proximity sensor, and the like.

The communication module 170 is connected to a network 30 by wire or wirelessly to communicate with an external device or a server. The communication module 170 may exchange data with a hospital server or another medical apparatus in a hospital, which is connected thereto via a PACS. Furthermore, the communication module 170 may perform data communication according to the digital imaging and communications in medicine (DICOM) standard.

The communication module 170 may transmit or receive data related to diagnosis of an object, e.g., an ultrasound image, ultrasound data, and Doppler data of the object, via the network 30 and may also transmit or receive medical images captured by another medical apparatus, e.g., a computed tomography (CT) apparatus, a magnetic resonance imaging (MRI) apparatus, or an X-ray apparatus. Furthermore, the communication module 170 may receive information about a diagnosis history or medical treatment schedule of a patient from a server and utilizes the received information to diagnose the patient. Furthermore, the communication module 170 may perform data communication not only with a server or a medical apparatus in a hospital, but also with a portable terminal of a medical doctor or patient.

The communication module 170 is connected to the network 30 by wire or wirelessly to exchange data with a server 32, a medical apparatus 34, or a portable terminal 36. The communication module 170 may include one or more components for communication with external devices. For example, the communication module 1300 may include a local area communication module 171, a wired communication module 172, and a mobile communication module 173.

The local area communication module 171 refers to a module for local area communication within a predetermined distance. Examples of local area communication techniques according to an embodiment may include, but are not limited to, wireless LAN, Wi-Fi, Bluetooth, ZigBee, Wi-Fi Direct (WFD), ultra wideband (UWB), infrared data association (IrDA), Bluetooth low energy (BLE), and near field communication (NFC).

The wired communication module 172 refers to a module for communication using electric signals or optical signals. Examples of wired communication techniques according to an embodiment may include communication via a twisted pair cable, a coaxial cable, an optical fiber cable, and an Ethernet cable.

The mobile communication module 173 transmits or receives wireless signals to or from at least one selected from a base station, an external terminal, and a server on a mobile communication network. The wireless signals may be voice call signals, video call signals, or various types of data for transmission and reception of text/multimedia messages.

The memory 180 stores various data processed by the ultrasound diagnosis apparatus 100. For example, the memory 180 may store medical data related to diagnosis of an object, such as ultrasound data and an ultrasound image that are input or output, and may also store algorithms or programs which are to be executed in the ultrasound diagnosis apparatus 100.

The memory 180 may be any of various storage media, e.g., a flash memory, a hard disk drive, EEPROM, etc. Furthermore, the ultrasound diagnosis apparatus 100 may utilize web storage or a cloud server that performs the storage function of the memory 180 online.

The input device 190 generates input data for controlling an operation of the ultrasound diagnosis apparatus 100. The input device 190 may include hardware components, such as a keypad, a mouse, a touch pad, a track ball, and a jog switch, but is not limited thereto. The input device 190 may further include any of various other components including an electrocardiogram (ECG) measuring module, a respiration measuring module, a voice recognition sensor, a gesture recognition sensor, a fingerprint recognition sensor, an iris recognition sensor, a depth sensor, a distance sensor, etc.

In particular, the input device 190 may also include a touch screen in which a touch pad forms a layer structure with the display 160.

In this case, according to an embodiment, the ultrasound diagnosis apparatus 100 may display an ultrasound image in a predetermined mode and a control panel for the ultrasound image on a touch screen. The ultrasound diagnosis apparatus 100 may also detect a user's touch gesture performed on an ultrasound image via the touch screen.

According to an embodiment, the ultrasound diagnosis apparatus 100 may include some buttons that are frequently used by a user among buttons that are included in a control panel of a general ultrasound apparatus, and provide the remaining buttons in the form of a GUI via a touch screen.

The controller 195 may control all operations of the ultrasound diagnosis apparatus 100. In other words, the controller 195 may control operations among the probe 20, the ultrasound transceiver 100, the image processor 150, the communication module 170, the memory 180, and the input device 190 shown in FIG. 1.

All or some of the probe 20, the ultrasound transceiver 115, the image processor 150, the communication module 170, the memory 180, the input device 190, and the controller 195 may be implemented as software modules. However, embodiments of the present invention are not limited thereto, and some of the components stated above may be implemented as hardware modules. Furthermore, at least one selected from the ultrasound transceiver 115, the image processor 150, and the communication module 170 may be included in the controller 195. However, embodiments of the present invention are not limited thereto.

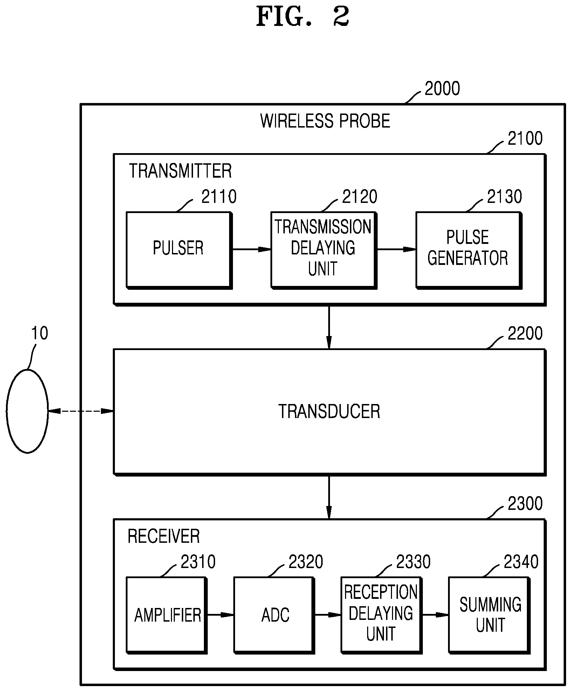

FIG. 2 is a block diagram showing a configuration of a wireless probe 2000 according to an embodiment. As described above with reference to FIG. 1, the wireless probe 2000 may include a plurality of transducers, and, according to embodiments, may include some or all of the components of the ultrasound transceiver 100 shown in FIG. 1.

The wireless probe 2000 according to the embodiment shown in FIG. 2 includes a transmitter 2100, a transducer 2200, and a receiver 2300. Since descriptions thereof are given above with reference to FIG. 1, detailed descriptions thereof will be omitted here. In addition, according to embodiments, the wireless probe 2000 may selectively include a reception delaying unit 2330 and a summing unit 2340.

The wireless probe 2000 may transmit ultrasound signals to the object 10, receive echo signals from the object 10, generate ultrasound data, and wirelessly transmit the ultrasound data to the ultrasound diagnosis apparatus 1000 shown in FIG. 1.

The wireless probe 2000 may be a smart device including a transducer array that is capable of performing an ultrasound scan. In detail, the wireless probe 2000 is a smart device that acquires ultrasound data by scanning an object via the transducer array. Then, the wireless probe 2000 may generate an ultrasound image by using the acquired ultrasound data and/or display the ultrasound image. The wireless probe 2000 may include a display via which a screen including at least one ultrasound image and/or a user interface screen for controlling an operation of scanning an object may be displayed.

While the user is scanning a predetermined body part of a patient that is an object by using the wireless probe 2000, the wireless probe 2000 and the ultrasound diagnosis apparatus 100 may continue to transmit or receive certain data therebetween via a wireless network. In detail, while the user is scanning a predetermined body part of a patient that is an object by using the wireless probe 2000, the wireless probe 2000 may transmit ultrasound data to the ultrasound diagnosis apparatus 100 in real-time via the wireless network. The ultrasound data may be updated in real-time as an ultrasound scan continues and then be transmitted from the wireless probe 2000 to the ultrasound diagnosis apparatus 100.

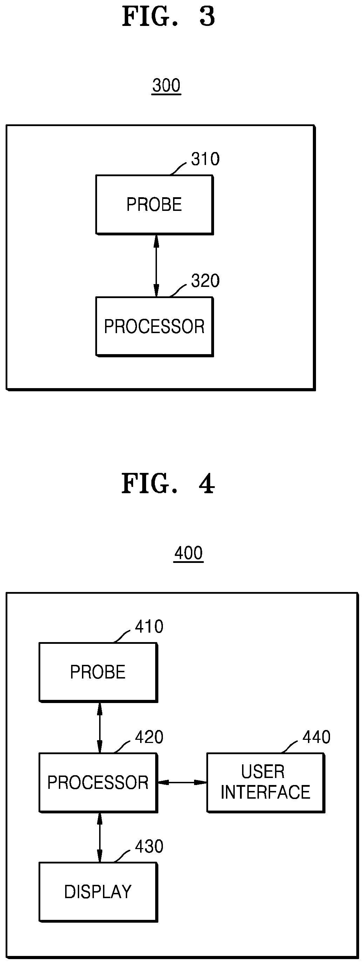

FIG. 3 is a block diagram of a configuration of an ultrasound imaging apparatus 300 according to an embodiment.

Referring to FIG. 3, the ultrasound imaging apparatus 300 according to the present embodiment may include a probe 310 and a processor 320. However, all of the components shown in FIG.3 are not essential components. The ultrasound imaging apparatus 300 may include more or fewer components than those shown in FIG. 3.

The probe 310 may include a plurality of transducers that convert an ultrasound signal into an electrical signal or vice versa. In other words, the probe 310 may include a transducer array consisting of a plurality of transducers. The plurality of transducers may be arranged in a one-dimensional (1D) or 2D array, and each of the plurality of transducers generates ultrasound signals separately or simultaneously. An ultrasound signal transmitted by each transducer is reflected off a discontinuous impedance surface within the object. Each transducer may convert a received reflected echo signal into an electrical reception signal.

The probe 310 may transmit an ultrasound signal to an object along a first path and receive an echo signal reflected from the object. In this case, the first path may be determined based on information about a position of an origin of an ultrasound beam composed of ultrasound signals and information about a transmission direction of the ultrasound beam. Furthermore, the information about a transmission direction of the ultrasound beam may be information about an angle between the transmission direction of the ultrasound beam and a transducer array.

The processor 320 may acquire first ultrasound data with respect to an object from reflected echo signals and generate a first ultrasound image based on the first ultrasound data. The processor 320 may detect at least one region having low image quality among regions in the first ultrasound image according to a predetermined criterion.

For example, when a region having low image quality is detected in the first ultrasound image according to the predetermined criterion, dual apodization with cross-correlation (DAX) may be used. In detail, if a correlation value for a first focal point, which is acquired using different apodization functions, is less than a predetermined threshold value, a region in the first ultrasound image corresponding to the first focal point may be detected as a region having low image quality. A point where information about an ultrasound image is to be acquired is referred to as a focal point.

More specifically, the ultrasound imaging apparatus 300 may generate lines RX1 and RX2 by applying different apodization functions to an echo signal. The ultrasound imaging apparatus 300 may calculate a DAX correlation by performing an arithmetic operation on the lines RX1 and RX2 according to Math Figure 1 below:

*Math Figure 1*

$$p(i,j) = \frac{\sum\limits_{k=i-A}^{i+A} RX1(k,j)RX2(k,j)}{\sqrt{\sum\limits_{k=i-A}^{i+A} RX1(k,j)^2}\sqrt{\sum\limits_{k=i-A}^{i+A} RX2(k,j)^2}}$$

where i and j represent a sample and a beam, respectively.

Furthermore, when a region having low image quality is detected in the first ultrasound image according to the predetermined criterion, a ratio representing consistency may be used. The ratio may be calculated by using Math Figure 2 below:

*Math Figure 2*

$$\left| \sum S_n(t) \right|^2 / \sum \left| S_n(t) \right|^2$$

where $S_n(t)$ denotes delayed ultrasound data for channel n.

The above-described predetermined criterion is merely an example, and it will be apparent to those of ordinary skill in the art that a region having low image quality may be detected in the first ultrasound image according to other criteria.

The probe 310 may transmit an ultrasound beam composed of ultrasound signals to an object in a plurality of directions and receive echo signals respectively reflected from the object based on the plurality of directions. By using the echo signals reflected from the object, the processor 320 may detect at least one region having low image quality among regions in the first ultrasound image according to a predetermined criterion.

The processor 320 may control an ultrasound signal to be transmitted along a second path based on the at least one region detected as a region having low image quality. The second path is different from the first path. Furthermore, transmission of an ultrasound signal along the first path may be achieved by focusing the ultrasound signal at a first focal point. Transmission of an ultrasound signal along the second path may be achieved by focusing the ultrasound signal at a second focal point.

The processor 320 may generate a second ultrasound image of an object based on an echo signal generated in response to an ultrasound signal transmitted to the object along the second path.

The processor 320 may control an ultrasound signal to be transmitted along a third path based on at least one region detected as a region having low image quality. The processor 320 may generate the second ultrasound image of the object based on the echo signals respectively generated in response to the ultrasound signals transmitted to the object along the second and third paths.

The processor 320 may control the probe 310 to perform beamforming by using a predetermined number of sub-apertures into which the plurality of transducers in the probe 310 are split. The beamforming is a process of increasing the intensity of ultrasound signals by overlapping the ultrasound signals during transmission and reception thereof via the plurality of transducers.

The ultrasound imaging apparatus 300 may obtain an ultrasound image by using another spatial path in order to improve an image quality of a region having low image quality in the ultrasound image.

The ultrasound imaging apparatus 300 may include a central arithmetic processor that controls overall operations of the probe 310 and the processor 320. The central arithmetic processor may be implemented as an array of a plurality of logic gates or a combination of a general purpose microprocessor and a program that can be run on the general purpose microprocessor. Furthermore, it will be appreciated by those of ordinary skill in the art to which the present embodiment pertains that the central arithmetic processor may be formed by different types of hardware.

FIG. 4 is a block diagram of a configuration of an ultrasound imaging apparatus 400 according to another embodiment.

Referring to FIG. 4, the ultrasound imaging apparatus 400 according to the present embodiment may include a probe 410, a processor 420, a display 430, and a user interface 440.

Since the probe 410 and the processor 420 of the ultrasound imaging apparatus 400 of FIG. 4 respectively correspond to the probe 310 and the processor 320 of the ultrasound imaging apparatus 300 of FIG. 3, descriptions that are already provided with respect to FIG. 3 will be omitted below. The ultrasound imaging apparatus 400 may include more or fewer components than those shown in FIG. 4.

The display 430 may display a predetermined screen. In detail, the display 430 may display a predetermined screen according to control by the processor 420. The display 430 includes a display panel (not shown) and displays a screen of the user interface 440, a medical image screen, etc. on the display panel.

The display 430 may display at least one of first and second ultrasound images. In this case, the first ultrasound image is generated based on an echo signal generated in response to an ultrasound signal transmitted to an object along a first path. The second ultrasound image is generated based on an echo signal generated in response to an ultrasound signal transmitted to the object along a second path. The second path is set to improve image quality of a region having low image quality in the first ultrasound image.

The processor 420 may detect at least one region having low image quality among regions in the first ultrasound image according to a predetermined criterion. The display 430 may display a map representing the quality of the first ultrasound image based on the detected at least one region.

The display 430 may display a map by distinguishing the at least one region from the other regions. For example, the display 430 may display a region having low image quality by using a red color and a region having appropriately high image quality by using a green color. Furthermore, the display 430 may display a boundary of a region having low image quality as a dashed or thick solid line in such a manner as to distinguish the region having the low image quality from a region having the appropriately high image quality.

The user interface 440 refers to a device via which a user inputs data for controlling the ultrasound imaging apparatus 400. The user interface 440 may include hardware components, such as a keypad, a mouse, a touch pad, a track ball, and a jog switch, but is not limited thereto. Furthermore, the user interface 440 may further include any of various other input tools including a voice recognition sensor, a gesture recognition sensor, an iris recognition sensor, a depth sensor, a distance sensor, etc.

The user interface 440 may receive a user input for transmitting an ultrasound signal along the second path based on a region having low image quality. The processor 420 may control an ultrasound signal to be transmitted along the second path based on the user input.

The user interface 440 may generate and output a user interface screen for receiving a predetermined command or data from the user. For example, the user interface 440 may generate and output a screen for setting at least one of an input for setting a position of an origin of an ultrasound beam in a map representing the quality of the first ultrasound image and an input for setting information about a transmission direction of the ultrasound beam.

The ultrasound imaging apparatus 400 may further include a storage device (not shown) and a communication module (not shown). The storage device and the communication module may respectively correspond to the memory 180 and the communication module 170 described with reference to FIG. 1. The storage device may store data related to an ultrasound image (e.g., an ultrasound image, ultrasound data, scan-related data, data related to diagnosis of a patient, etc.), data transmitted from an external device to the ultrasound imaging apparatus 400, etc. The data transmitted from the external device may include patient-related information, data necessary for diagnosis and treatment of a patient, a patient's past medical history, a medical work list corresponding to instructions regarding diagnosis of a patient, and the like.

The communication module may receive and/or transmit data from and/or to an external device. For example, the communication module may connect to a wireless probe or an external device via a communication network based on Wi-Fi or Wi-Fi Direct (WFD) technology. In detail, examples of a wireless communication network to which the communication module can connect may include, but are not limited to, Wireless LAN (WLAN), Wi-Fi, Bluetooth, ZigBee, WFD, Ultra Wideband (UWB), Infrared Data Association (IrDA), Bluetooth Low Energy (BLE), and Near Field Communication (NFC).

The ultrasound imaging apparatus 400 may include a central arithmetic processor that controls overall operations of the probe 410, the processor 420, the display 430, the user interface 440, the storage device, and the communication module. The central arithmetic processor may be implemented as an array of a plurality of logic gates or a combination of a general purpose microprocessor and a program that can be run on the general purpose microprocessor. Furthermore, it will be appreciated by those of ordinary skill in the art to which the present embodiment pertains that the central arithmetic processor may be formed by different types of hardware.

Hereinafter, various operations performed by the ultrasound imaging apparatus 300 (400) and applications thereof will be described in detail. Although none of the probe 310 (410), the processor 320 (420), the display 430, the user interface 440, the storage device, and the communication module are specified, features and aspects that would be clearly understood by and are obvious to those of ordinary skill in the art may be considered as a typical implementation. The scope of the present inventive concept is not limited by a name of a particular component or physical/logical structure.

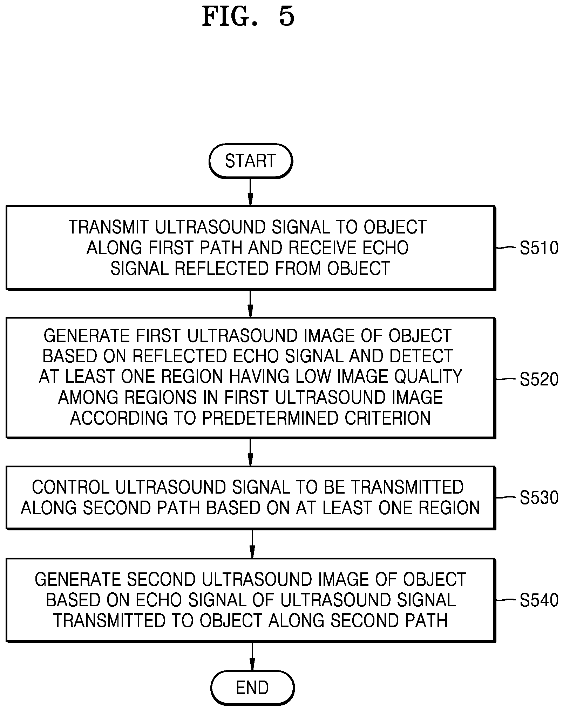

FIG. 5 is a flowchart of a method of operating an ultrasound imaging apparatus according to an embodiment.

Referring to FIG. 5, the ultrasound imaging apparatus may transmit an ultrasound signal to an object along a first path and receive an echo signal reflected from the object (S510). In this case, the first path may be determined based on information about a position of an origin of an ultrasound beam composed of ultrasound signals and information about a transmission direction of the ultrasound beam. Furthermore, the information about a transmission direction of the ultrasound beam may be information about an angle between the transmission direction of the ultrasound beam and a transducer array.

The ultrasound imaging apparatus may generate a first ultrasound image of the object based on the reflected echo signal and detect at least one region having low image quality among regions in the first ultrasound image according to a predetermined criterion (S520).

In this case, if a correlation value for a first focal point, which is acquired using different apodization functions, is less than a predetermined threshold value, a region in the first ultrasound image corresponding to the first focal point may be detected as a region having low image quality.

Furthermore, the ultrasound imaging apparatus may transmit an ultrasound beam to the object in a plurality of directions and receive echo signals respectively reflected from the object based on the plurality of directions. By using the echo signals, the ultrasound imaging apparatus may detect at least one region having low image quality among regions in the first ultrasound image according to a predetermined criterion.

The ultrasound imaging apparatus may control an ultrasound signal to be transmitted along a second path based on the detected at least one region (S530). Furthermore, to improve an image quality of a region having low image quality, the ultrasound imaging apparatus may control an ultrasound signal to be transmitted along a third path, which is different from the second path, based on the detected at least one region.

The ultrasound imaging apparatus may generate a second ultrasound image of the object based on an echo signal generated in response to the ultrasound signal transmitted to the object along the second path (S540).

The ultrasound imaging apparatus may generate a second ultrasound image of the object based on the echo signals respectively generated in response to the ultrasound signals transmitted to the object along the second and third paths.

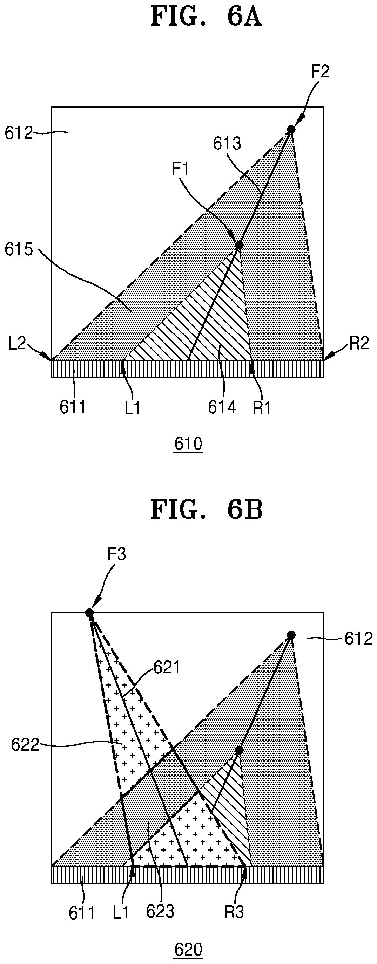

FIG. 6A is a diagram for explaining detection of a region having low image quality among regions in an ultrasound image, according to an embodiment.

An ultrasound imaging apparatus may generate a first ultrasound image based on an echo signal generated in response to an ultrasound signal transmitted to an object along a first path. In the presence of factors that degrade quality of the first ultrasound image, the quality of the first ultrasound image needs to be improved. The factors that degrade the quality of the first ultrasound image may be bone, fibrous tissue, adipose tissue, etc., but are not limited thereto. Since the factors are present in the object and cannot be removed directly, it is necessary to generate an ultrasound image without being affected much by the factors. The ultrasound imaging apparatus may detect a region having low image quality among regions in the first ultrasound image and generate a map representing a quality of the first ultrasound image based on the detected region.

FIG. 6A is a diagram for explaining a process of generating a map indicating the quality of an ultrasound image by detecting a region having low image quality among regions in the ultrasound image.

Referring to 610 of FIG. 6A, a transducer 611 of the ultrasound imaging apparatus may transmit an ultrasound signal to the object along a first path 613 and receive an echo signal that is focused at a first focal point F1 in an imaging area 612 by using an aperture from L1 to R1. Furthermore, the ultrasound imaging apparatus may receive an echo signal that is focused at a second focal point F2 in the imaging area 612 by using an aperture from L2 to R2.

In detail, for example, the ultrasound imaging apparatus may detect a region having low image quality in the first ultrasound image by using a DAX correlation. The ultrasound imaging apparatus may calculate a DAX correlation value for the first focal point F1 and a DAX correlation value for the second focal point F2 respectively by using different apodization functions. If the DAX correlation value for the first focal point F1 is close to or less than 1 by a predetermined value, the ultrasound imaging apparatus may determine a region 614 as having appropriately high image quality. Furthermore, if the DAX correlation value for the second focal point F2 is close to or less than 0, the ultrasound imaging apparatus may determine a region 615 as having low image quality.

FIG. 6B is a diagram for explaining detection of a region having low image quality among regions in an ultrasound image, according to another embodiment.

Referring to 620 of FIG. 6B, the transducer 611 of the ultrasound imaging apparatus may transmit an ultrasound signal to the object along a path 621 that is different from the first path 613 and receive an echo signal that is focused at a third focal point F3 in the imaging area 612 by using an aperture from L3 to R3. If a DAX correlation value for the third focal point F3 is close to or less than 1 by a predetermined value, the ultrasound imaging apparatus may determine a region 622 as being a region having appropriately high image quality. Furthermore, if a DAX correlation value for the third focal point F3 is close to or less than 0, the ultrasound imaging apparatus may determine a region 623 as being a region having low image quality.

The ultrasound imaging apparatus may transmit an ultrasound beam composed of ultrasound signals to the object in a plurality of directions and receive echo signals respectively reflected from the object based on the plurality of directions. The ultrasound imaging apparatus may detect a region having low image quality among regions in the first ultrasound image by using the reflected echo signals. By transmitting an ultrasound beam in a plurality of directions and receiving reflected echo signals, the ultrasound imaging apparatus may detect a region having low image quality in an ultrasound image more accurately and efficiently.

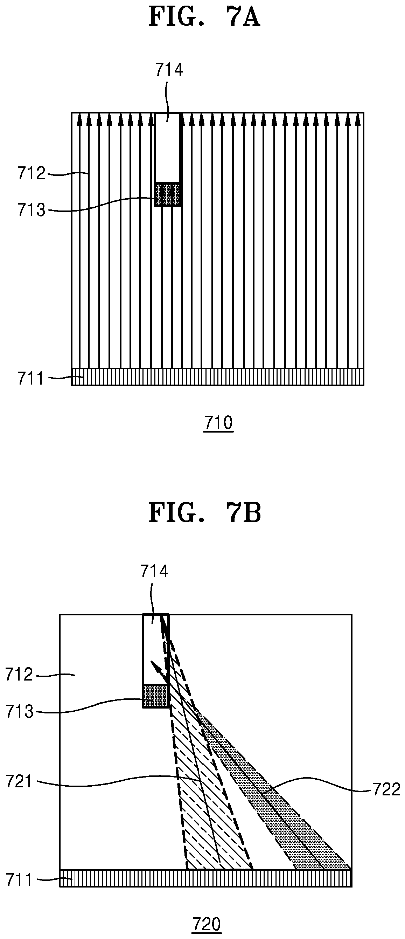

FIG. 7A is a diagram for explaining a factor that degrades the quality of an ultrasound image, according to an embodiment.

Referring to FIG. 7A, a transducer 711 may transmit an ultrasound signal to an object and receive an echo signal reflected from the object. When a factor 713 such as bone, fibrous tissue, or fat is in the object, an ultrasound signal cannot reach a far end inside the object. Thus, the ultrasound imaging apparatus is not able to receive an echo signal reflected from a region 714 within an imaging area 712. If the ultrasound imaging apparatus generates a first ultrasound image 710 of the object without receiving the echo signal reflected from the region 714, an image quality of the region 714 may be degraded. Thus, the ultrasound imaging apparatus may acquire a location of the factor 713 that degrades image quality by using a DAX correlation. The ultrasound imaging apparatus may transmit an ultrasound signal to the object based on the location of the factor 713.

FIG. 7B is a diagram for explaining a method of improving the quality of an ultrasound image according to an embodiment.

The ultrasound imaging apparatus may transmit an ultrasound beam to a region of interest (ROI) of an object in a plurality of directions and receive echo signals respectively reflected from the ROI of the object based on the plurality of directions. By using the echo signals reflected from the object, the ultrasound imaging apparatus may detect a region having low image quality among regions in a first ultrasound image according to a predetermined criterion.

When the ultrasound imaging apparatus transmits an ultrasound beam in directions that are perpendicular to the transducer 711, as shown in FIG. 7A, the ultrasound imaging apparatus is not able to receive echo signals reflected from the region 714 within the imaging area 712 due to the presence of the factor 713 (i.e., an obstacle) in the object. The ultrasound imaging apparatus may generate the first ultrasound image 710 based on echo signals reflected from the other regions excluding the region 714 and detect the region 714 having low image quality among regions in the first ultrasound image 710 according to a predetermined criterion.

As shown in FIG. 7B, the ultrasound imaging apparatus may determine second paths 721 and 722 so that ultrasound signals reach a region 714 without being obstructed by a factor 713 that degrades an image quality. In detail, the ultrasound imaging apparatus may determine a range of an aperture so as to transmit an ultrasound signal by focusing the ultrasound signal at a focal point within the region 714 having low image quality and to receive an echo signal without being disturbed by the factor 713 that degrades the image quality. The ultrasound imaging apparatus may transmit ultrasound signals to the region 714 along the second paths 721 and 722 and receive echo signals reflected from the region 714. The ultrasound imaging apparatus may generate a second ultrasound image 720 by using ultrasound data acquired from the echo signals.

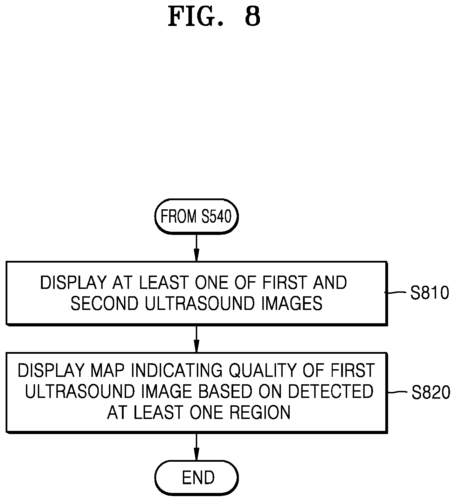

FIG. 8 is a flowchart of a method of operating an ultrasound imaging apparatus according to another embodiment.

Referring to FIG. 8, the ultrasound imaging apparatus may display at least one of the first and second ultrasound images (S810). In this case, the first ultrasound image is generated based on the echo signal received in response to the ultrasound signal transmitted to the object along the first path. The second ultrasound image is generated based on the echo signal received in response to the ultrasound signal transmitted to the object along the second path. The second path is set to improve an image quality of the region having low image quality in the first ultrasound image.

The ultrasound imaging apparatus may display a map indicating a quality of the first ultrasound image based on the region detected as a region having low image quality (S820). According to an embodiment, after performing operation S540, the ultrasound imaging apparatus may perform operation S820 by skipping operation S810.

The ultrasound imaging apparatus may display a region having low image quality and a region having appropriately high image quality in the map in such a manner as to distinguish them from each other. For example, the regions having low image quality and having appropriately high image quality may be displayed using different colors. Furthermore, a boundary of the region having low image quality may be displayed as at least one of a solid line, a thick solid line, a dashed line, and a thick dashed line, and embodiments are not limited thereto.

Furthermore, the ultrasound imaging apparatus may display the first ultrasound image together with the map indicating the quality of the first ultrasound image. Furthermore, the ultrasound imaging apparatus may display at least one of the first ultrasound image, the second ultrasound image, and the map indicating the quality of the first ultrasound image.

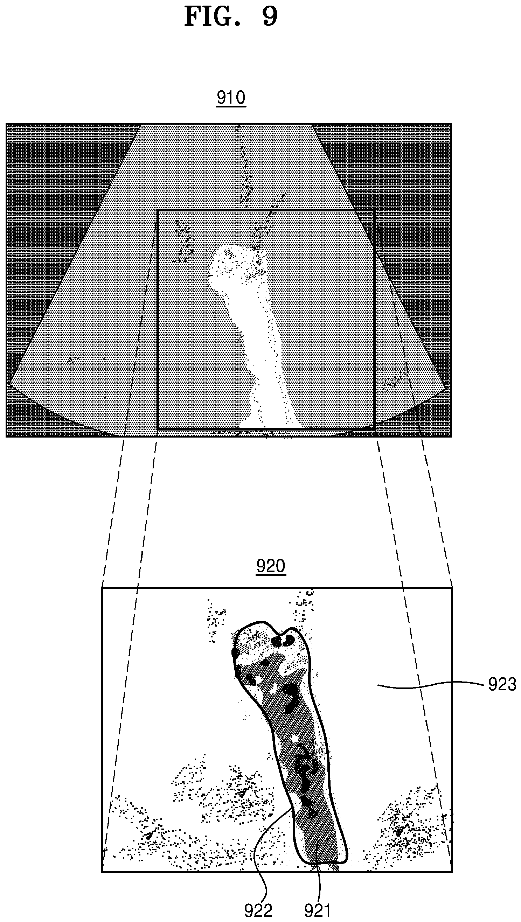

FIG. 9 is diagram for explaining a first ultrasound image and a map indicating a quality of the first ultrasound image, according to an embodiment.

An ultrasound imaging apparatus generates an ultrasound image based on ultrasound data. A plurality of modes for providing an ultrasound image (hereinafter, referred to as 'composite mode') may include a B-mode for providing a B-mode image, a Color Doppler mode (C-mode) or a Power Doppler mode (P-mode) for providing a color flow image, and a D- mode for providing a Doppler spectrum. The ultrasound imaging apparatus may display via a screen of a display an ultrasound image in one of the plurality of modes.

Referring to FIG. 9, the ultrasound imaging apparatus may transmit an ultrasound signal to a woman's uterus and acquire ultrasound data by receiving an echo signal reflected from the woman's uterus. The ultrasound imaging apparatus may generate a first ultrasound image 910 based on the acquired ultrasound data. The ultrasound imaging apparatus may also display the first ultrasound image 910 via a screen of the display. In this case, the first ultrasound image 910 may be a B-mode image showing the myometrium and endometrium of the uterus. In addition, due to calcification of the myometrium and endometrium, the quality of the first ultrasound image 910 may decrease away from a region where calcification occurs.

The ultrasound imaging apparatus may detect a region having low image quality among regions in the first ultrasound image 910 according to a predetermined criterion. The ultrasound imaging apparatus may display a map 920 indicating a quality of the first ultrasound image 910 in such a manner as to distinguish a region having low image quality from the other regions.

For example, in the map 920, the ultrasound imaging apparatus may display a region 921 having low image quality by using dark colors while displaying a region 923 having appropriately high image quality by using bright colors. Furthermore, the ultrasound imaging apparatus may display a boundary 922 of the region 921 having low image quality as one of a solid line, a thick solid line, a dashed line, and a thick dashed line. Furthermore, the ultrasound imaging apparatus may display the boundary 922 of the region 921 by using a red color. It will be understood by those of ordinary skill in the art that the ultrasound imaging apparatus may display the map 920 in such a manner as to distinguish the region 921 having low image quality from the region 923 having appropriately high image quality by using methods other than those described above.

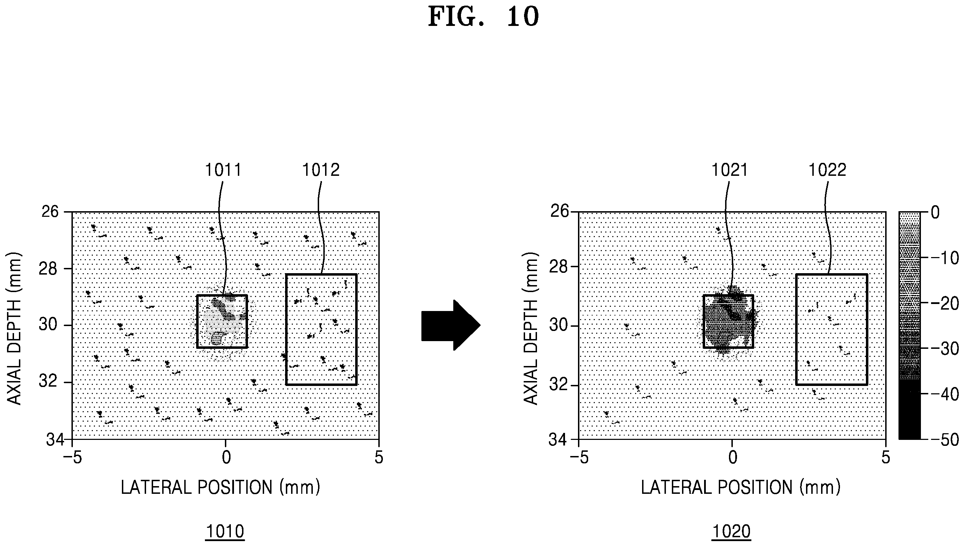

FIG. 10 is a diagram for explaining a first ultrasound image obtained before undergoing improvement of image quality and a second ultrasound image obtained after undergoing improvement of image quality, according to an embodiment.

When a factor that obstructs a path of an ultrasound signal exists in an object, a quality of an ultrasound image may be degraded due to the presence of the factor. An image 1010 of FIG. 10 may be an ultrasound image obtained based on a reflected echo signal without taking into account a path of an ultrasound signal.

On the other hand, an image 1020 of FIG. 10 may be an ultrasound image obtained based on a reflected echo signal by taking into account a factor that obstructs a path of an ultrasound signal, i.e., by allowing the ultrasound signal to circumvent the factor and propagate into the entire area of the object.

By comparing the images 1010 and 1020 with each other, it can be seen that a region 1021 appears clearer than a region 1011 and that the number of black dots in a region 1022 is reduced compared to the number of black dots in a region 1012.

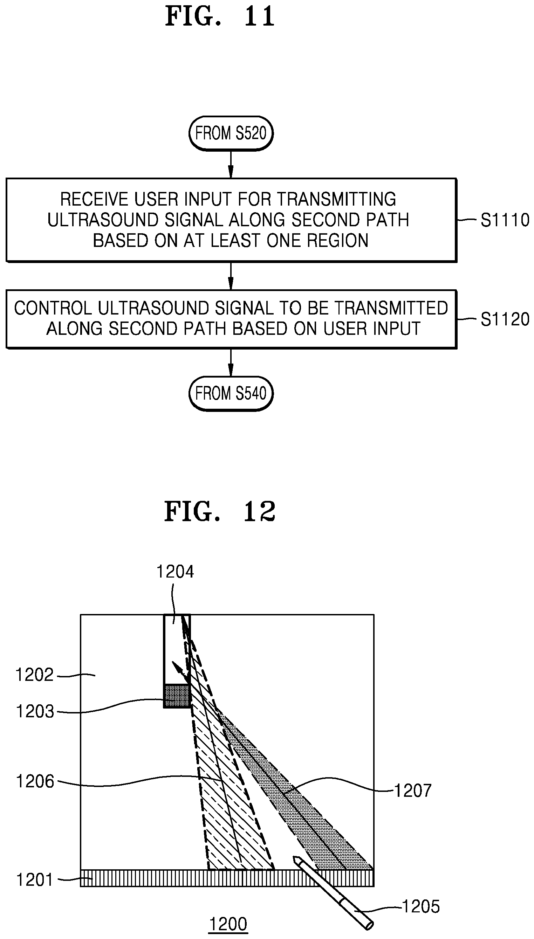

FIG. 11 is a flowchart of a method of operating an ultrasound imaging apparatus according to another embodiment.

Referring to FIG. 11, the ultrasound imaging apparatus may receive a user input for transmitting an ultrasound signal along a second path based on the detected at least one region (S1110). The ultrasound imaging apparatus may receive a user input for setting at least one piece of information from among information about a position of an origin of an ultrasound beam, information about a transmission direction of the ultrasound beam, and information about an aperture size. In this case, the user input may be received via a control panel, a track ball, a mouse, a keyboard, etc.

In detail, to determine the second path, the user may display the second path on a screen by using a keypad, a mouse, a touch screen, a track ball, a jog switch, etc.

The ultrasound imaging apparatus may control the ultrasound signal to be transmitted along the second path based on the user input (S1120).

FIG. 12 is a diagram for explaining a method of controlling an operation of an ultrasound imaging apparatus based on a user input, according to an embodiment.

Referring to FIG. 12, the ultrasound imaging apparatus may generate and output a user interface screen 1200 for setting transmission of an ultrasound signal along a second path based on a region having low image quality.

A user interface may also receive a predetermined command or data from the user via the user interface screen 1200. For example, the user interface may receive, from the user, at least one of a position of an origin of an ultrasound beam and a transmission direction of the ultrasound beam. The user interface screen 1200 may receive a manipulation signal via a user's touch input by using various input tools. The user interface screen 1200 may receive an input for adjusting via a user's hand or physical tool a position of an origin of an ultrasound beam, a transmission direction of the ultrasound beam, an aperture size, etc., displayed on the user interface screen 1200.

In detail, the user may input a drag and drop signal for determining second paths 1206 and 1207 via a touch pen 1205 so that an ultrasound signal may reach a region 1204 without being obstructed by a factor 1203 that degrades an image quality of an imaging area 1202. A transducer 1201 of the ultrasound imaging apparatus may transmit ultrasound signals to the region 1204 along the second paths 1206 and 1207 and receive echo signals reflected from the region 1204. The ultrasound imaging apparatus may generate a second ultrasound image based on ultrasound data acquired from the echo signals.

The ultrasound imaging apparatuses described above may be implemented using hardware components, software components, and/or a combination thereof. For example, the apparatuses and components illustrated in the embodiments may be implemented using one or more general-purpose or special-purpose computers, such as a processor, a controller, an arithmetic logic unit (ALU), a digital signal processor, a microcomputer, a field programmable array (FPA), a programmable logic unit (PLU), a microprocessor, or any other device capable of responding to and executing instructions.

A processing device may run an operating system (OS) and one or more software applications running on the OS. The processing device also may access, store, manipulate, process, and create data in response to execution of software.

Although a single processing device may be illustrated for convenience, one of ordinary skill in the art will appreciate that a processing device may include a plurality of processing elements and/or a plurality of types of processing elements. For example, a processing device may include a plurality of processors or a processor and a controller. In addition, the processing device may have different processing configurations such as parallel processors.

Software may include a computer program, a piece of code, an instruction, or one or more combinations thereof and independently or collectively instruct or configure the processing device to operate as desired.

Software and/or data may be embodied permanently or temporarily in any type of machine, component, physical equipment, virtual equipment, computer storage medium or device, or in a transmitted signal wave so as to be interpreted by the processing device or to provide instructions or data to the processing device. The software also may be distributed over network-coupled computer systems so that the software is stored and executed in a distributed fashion. In particular, the software and data may be stored in one or more computer-readable recording media.

The methods according to the embodiments may be recorded in non-transitory computer-readable recording media including program instructions to implement various operations embodied by a computer. The non-transitory computer-readable recording media may also include, alone or in combination with the program instructions, data files, data structures, and the like. The program instructions recorded in the non-transitory computer-readable recording media may be designed and configured specially for the exemplary embodiments or be known and available to those of ordinary skill in computer software.

Examples of non-transitory computer-readable recording media include magnetic media such as hard disks, floppy disks, and magnetic tape, optical media such as CD-ROM discs and DVDs, magneto-optical media such as floptical discs, and hardware devices that are specially configured to store and perform program instructions, such as ROM, RAM, flash memory, and the like.

Examples of program instructions include both machine code, such as that produced by a compiler, and higher level code that may be executed by the computer using an interpreter.

The above-described hardware devices may be configured to act as one or more software modules in order to perform the operations of the above-described embodiments, or vice versa.

While one or more exemplary embodiments have been described with reference to the figures, it will be understood by those of ordinary skill in the art that various modifications and changes in form and details may be made from the above descriptions without departing from the scope as defined by the following claims.

Thus, the scope of the present inventive concept is defined not by the detailed description thereof but by the appended claims and their equivalents.

**Claims**

1. An ultrasound imaging apparatus comprising:

   a probe configured to transmit an ultrasound signal to an object along a first path by focusing the ultrasound signal at a first focal point and receive an echo signal reflected from the object; and
   a processor configured to generate a first ultrasound image representing the object, detect at least one region having low image quality among regions in the generated first ultrasound image according to a predetermined criterion,
   wherein the processor is configured to generate a map indicating quality of the first ultrasound image based on the detected at least one region, control the probe based on the map so that the ultrasound signal reaches the detected at least one region along a second path by focusing the ultrasound signal at a second focal point within a predetermined region of the object, wherein the second path is determined based on a factor obstructing the first path, and generate a second ultrasound image representing the object based on an echo signal received in response to the ultrasound signal transmitted to the object along the second path,
   wherein the second path is determined based on a change in at least one of a position of an origin of an ultrasound beam corresponding to the first path and a transmission direction of the ultrasound beam corresponding to the first path.

2. The ultrasound imaging apparatus of claim 1, wherein the probe is further configured to transmit an ultrasound beam composed of the ultrasound signal to the object in a plurality of directions and receive echo signals respectively reflected from the object based on the plurality of directions, and
   wherein the processor is further configured to detect, according to a predetermined criterion, at least one region having low image quality among regions in the first ultrasound image by using the reflected echo signals.

3. The ultrasound imaging apparatus of claim 1, wherein when detecting the at least one region having low image quality according to the predetermined criterion, the processor detects, if a correlation value for the first focal point, which is acquired using different apodization functions, is less than a predetermined threshold value, a region in the first ultrasound image corresponding to the first focal point as a region having low image quality.

4. The ultrasound imaging apparatus of claim 1, further comprising a display configured to display at least one of the first and second ultrasound images, and display the map indicating quality of the first ultrasound image based on the detected at least one region.

5. The ultrasound imaging apparatus of claim 1, wherein the probe comprises a transducer array consisting of a plurality of transducers, wherein the information about the transmission direction of the ultrasound beam is information about an angle between the transmission direction of the ultrasound beam and the transducer array.

6. The ultrasound imaging apparatus of claim 1, wherein the processor is further configured to control the ultrasound signal to be transmitted along a third path based on the at least one region and generate a second ultrasound image corresponding to the object based on an echo signal received in response to the ultrasound signal transmitted to the object along the second path and an echo signal received in response to the ultrasound signal transmitted to the object along the third path.

7. The ultrasound imaging apparatus of claim 1, further comprising a user interface configured to receive a user input for setting transmission of the ultrasound signal along the second path based on the at least one region, and
   wherein the processor is further configured to control the ultrasound signal to be transmitted along the second path based on the user input.

8. The ultrasound imaging apparatus of claim 1, wherein the processor is further configured to control the probe to perform beamforming by using a predetermined number of sub-apertures into which a plurality of transducers in the probe are divided.

9. A method of operating an ultrasound imaging apparatus, the method comprising:

transmitting, by a probe in the ultrasound imaging apparatus, an ultrasound signal to an object along a first path by focusing the ultrasound signal at a first focal point and receiving an echo signal reflected from the object;
generating a first ultrasound image representing the object and detecting at least one region having low image quality among regions in the generated first ultrasound image according to a predetermined criterion; and
generating a second ultrasound image representing the object based on an echo signal received in response to the ultrasound signal transmitted to the object along a second path,
wherein the generating the second ultrasound image comprises,
generating a map indicating quality of the first ultrasound image based on the detected at least one region; and
controlling the probe based on the map so that the ultrasound signal reaches the detected at least one region along the second path by focusing the ultrasound signal at a second focal point within a predetermined region of the object, wherein the second path is determined based on a factor obstructing the first path,
wherein the second path is determined based on a change in at least one of a position of an origin of an ultrasound beam corresponding to the first path and a transmission direction of the ultrasound beam corresponding to the first path.

10. The method of claim 9, wherein the transmitting of the ultrasound signal to the object along the first path and the receiving of the echo signal reflected from the object comprises transmitting an ultrasound beam composed of the ultrasound signal to the object in a plurality of directions and receiving echo signals respectively reflected from the object based on the plurality of directions, and
wherein the generating of the first ultrasound image and the detecting of the at least one region having low image quality according to the predetermined criterion comprises detecting, according to a predetermined criterion, at least one region having low image quality among regions in the first ultrasound image by using the reflected echo signals.

11. The method of claim 9, wherein the detecting of the at least one region having low image quality according to the predetermined criterion comprises detecting, if a correlation value for the first focal point, which is acquired using different apodization functions, is less than a predetermined threshold value, a region in the first ultrasound image corresponding to the first focal point as a region having low image quality.

12. The method of claim 9, further comprising displaying the second ultrasound images.


**Patentansprüche**

1. Ultraschallbildgebungsvorrichtung, die Folgendes umfasst:

eine Sonde, die dazu konfiguriert ist, ein Ultraschallsignal durch Bündeln des Ultraschallsignals an einem ersten Brennpunkt entlang einer ersten Bahn auf ein Objekt zu übertragen, und ein von dem Objekt reflektiertes Echosignal zu empfangen; und
einen Prozessor, der dazu konfiguriert ist, ein das Objekt darstellendes erstes Ultraschallbild zu erzeugen und gemäß einem vorbestimmten Kriterium wenigstens einen Bereich unter den Bereichen in dem ersten ersten Ultraschallbild zu erfassen, der eine niedrige Bildqualität aufweist,
wobei der Prozessor zu Folgendem konfiguriert ist: Erzeugen einer Karte, die eine Qualität des ersten Ultraschallbildes angibt, basierend auf dem erfassten wenigstens einen Bereich, Steuern der Sonde basierend auf der Karte, so dass das Ultraschallsignal den erfassten wenigstens einen Bereich entlang einer zweiten Bahn erreicht, durch Bündeln des Ultraschallsignals an einem zweiten Brennpunkt innerhalb eines vorbestimmten Bereichs des Objekts, wobei die zweite Bahn basierend auf einem Faktor, der die erste Bahn behindert, bestimmt wird, und Erzeugen eines das Objekt darstellenden zweiten Ultraschallbildes basierend auf einem Echosignal, das als Antwort auf das entlang einer zweiten Bahn auf das Objekt übertragene Ultraschallsignal empfangen wird,
wobei die zweite Bahn bestimmt wird basierend auf einer Veränderung bei der Position eines Ursprungs eines der ersten Bahn entsprechenden Ultraschallstrahls und/oder einer Übertragungsrichtung des der ersten Bahn entsprechenden Ultraschallstrahls.

**2.** Ultraschallbildgebungsvorrichtung nach Anspruch 1, wobei die Sonde weiterhin dazu konfiguriert ist, einen aus dem Ultraschallsignal bestehenden Ultraschallstrahl in einer Vielzahl von Richtungen auf das Objekt zu übertragen und jeweils basierend auf der Vielzahl von Richtungen von dem Objekt reflektierte Echosignale zu empfangen, und wobei der Prozessor weiterhin dazu konfiguriert ist, gemäß einem vorbestimmten Kriterium wenigstens einen Bereich unter den Bereichen in dem ersten Ultraschallbild zu erfassen, der eine niedrige Bildqualität aufweist, und zwar unter Verwendung des reflektierten Echosignals.

**3.** Ultraschallbildgebungsvorrichtung nach Anspruch 1, wobei, wenn der wenigstens eine Bereich, der eine niedrige Bildqualität aufweist, gemäß dem vorbestimmten Kriterium erfasst wird, der Prozessor einen dem ersten Brennpunkt entsprechenden Bereich in dem ersten Ultraschallbild als einen Bereich mit niedriger Bildqualität erfasst, wenn ein Korrelationswert für den ersten Brennpunkt, der unter Verwendung verschiedener Apodisationsfunktionen erhalten wird, kleiner als ein vorbestimmter Schwellenwert ist.

**4.** Ultraschallbildgebungsvorrichtung nach Anspruch 1, die weiterhin eine Anzeige umfasst, die dazu konfiguriert ist, das erste und/oder das zweite Ultraschallbild anzuzeigen und die Karte anzuzeigen, die eine Qualität des ersten Ultraschallbildes basierend auf dem erfassten wenigstens einen Bereich angibt.

**5.** Ultraschallbildgebungsvorrichtung nach Anspruch 1, wobei die Sonde eine Wandleranordnung umfasst, die aus einer Vielzahl von Wandlern besteht, wobei es sich bei Informationen über die Übertragungsrichtung des Ultraschallstrahls um Informationen über einen Winkel zwischen der Übertragungsrichtung des Ultraschallstrahls und der Wandleranordnung handelt.

**6.** Ultraschallbildgebungsvorrichtung nach Anspruch 1, wobei der Prozessor weiterhin dazu konfiguriert ist, das Ultraschallsignal derart zu steuern, dass es basierend auf dem wenigstens einen Bereich entlang einer dritten Bahn übertragen wird, und ein dem Objekt entsprechendes zweites Ultraschallbild basierend auf einem Echosignal, das als Antwort auf das entlang der zweiten Bahn auf das Objekt übertragene Ultraschallsignal empfangen wurde, und einem Echosignal, das als Antwort auf das entlang der dritten Bahn auf das Objekt übertragene Ultraschallsignal empfangen wurde, zu erzeugen.

**7.** Ultraschallbildgebungsvorrichtung nach Anspruch 1, die weiterhin eine Benutzeroberfläche umfasst, die dazu konfiguriert ist, eine Benutzereingabe zur Einstellung der Übertragung des Ultraschallsignals entlang der zweiten Bahn basierend auf dem wenigstens einen Bereich zu empfangen, und wobei der Prozessor weiterhin dazu konfiguriert ist, das Ultraschallsignal derart zu steuern, dass es basierend auf der Benutzereingabe entlang der zweiten Bahn übertragen wird.

**8.** Ultraschallbildgebungsvorrichtung nach Anspruch 1, wobei der Prozessor weiterhin dazu konfiguriert ist, die Sonde derart zu steuern, dass sie durch Verwenden einer vorbestimmten Anzahl von Neben-Blenden, in die eine Vielzahl von Wandlern in der Sonde unterteilt ist, eine Strahlformung durchführt.

**9.** Verfahren zum Betrieb einer Ultraschallbildgebungsvorrichtung, wobei das Verfahren Folgendes umfasst:

durch eine Sonde in der Ultraschallbildgebungsvorrichtung, Übertragen eines Ultraschallsignals entlang einer ersten Bahn auf ein Objekt durch Bündeln des Ultraschallsignals an einem ersten Brennpunkt, und Empfangen eines von dem Objekt reflektierten Echosignals;
Erzeugen eines das Objekt darstellenden ersten Ultraschallbildes und Erfassen wenigstens eines Bereichs unter den Bereichen in dem ersten ersten Ultraschallbild, der eine niedrige Bildqualität aufweist, gemäß einem vorbestimmten Kriterium; und
Erzeugen eines das Objekt darstellenden zweiten Ultraschallbildes basierend auf einem Echosignal, das als Antwort auf das entlang einer zweiten Bahn auf das Objekt übertragene Ultraschallsignal empfangen wird, wobei das Erzeugen des zweiten Ultraschallbildes Folgendes umfasst:

Erzeugen einer Karte, die eine Qualität des ersten Ultraschallbildes angibt, basierend auf dem erfassten wenigstens einen Bereich; und
Steuern der Sonde basierend auf der Karte derart, dass das Ultraschallsignal den erfassten wenigstens einen Bereich entlang der zweiten Bahn erreicht durch Bündeln des Ultraschallsignals an einem zweiten Brennpunkt innerhalb eines vorbestimmten Bereichs des Objekts, wobei die zweite Bahn basierend auf einem Faktor, der die erste Bahn behindert, bestimmt wird, wobei die zweite Bahn bestimmt wird basierend auf einer Veränderung bei der Position eines Ursprungs eines der ersten Bahn entsprechenden Ultraschallstrahls und/oder einer Übertragungsrichtung des der ersten Bahn entsprechenden Ultraschallstrahls.

10. Verfahren nach Anspruch 9, wobei das Übertragen des Ultraschallsignals an das Objekt entlang der ersten Bahn und das Empfangen des von dem Objekt reflektierten Echosignals Folgendes umfassen: Übertragen eines aus dem Ultraschallsignal bestehenden Ultraschallstrahls in einer Vielzahl von Richtungen auf das Objekt und Empfangen der jeweils basierend auf der Vielzahl von Richtungen von dem Objekt reflektierten Echosignale, und wobei das Erzeugen des ersten Ultraschallbildes und das Erfassen des wenigstens einen, eine niedrige Bildqualität aufweisenden Bereichs gemäß dem vorbestimmten Kriterium Folgendes umfasst: gemäß einem vorbestimmten Kriterium, Erfassen wenigstens eines Bereichs unter den Bereichen in dem ersten Ultraschallbild, der eine niedrige Bildqualität aufweist, und zwar unter Verwendung der reflektierten Echosignale.

11. Verfahren nach Anspruch 9, wobei das Erfassen des wenigstens einen Bereichs, der eine niedrige Bildqualität aufweist, gemäß dem vorbestimmten Kriterium, Folgendes umfasst: wenn ein Korrelationswert für den ersten Brennpunkt, der unter Verwendung verschiedener Apodisationsfunktionen erhalten wird, kleiner als ein vorbestimmter Schwellenwert ist, Erfassen eines Bereichs in dem ersten Ultraschallbild, der dem ersten Brennpunkt entspricht, als einen Bereich mit niedriger Bildqualität.

12. Verfahren nach Anspruch 9, das weiterhin ein Anzeigen des zweiten Ultraschallbildes umfasst.

**Revendications**

1. Appareil échographique comprenant :

    une sonde conçue pour émettre un signal ultrasonore en direction d'un objet le long d'une première trajectoire en concentrant le signal ultrasonore en un premier foyer et pour recevoir un signal d'écho réfléchi par l'objet, et un processeur conçu pour générer une première image échographique représentant l'objet, détecter au moins une région offrant une faible qualité d'image parmi les régions présentes dans la première image échographique générée conformément à un critère prédéterminé ;
    ledit processeur étant conçu pour générer une carte indicatrice de la qualité de la première image échographique compte tenu de l'au moins une région détectée, pour commander la sonde compte tenu de la carte de façon que le signal ultrasonore parvienne à l'au moins une région détectée le long d'une deuxième trajectoire en concentrant le signal ultrasonore en un deuxième foyer au sein d'une région prédéterminée de l'objet, ladite deuxième trajectoire étant déterminée compte tenu d'un facteur obstruant la première trajectoire, et pour générer une deuxième image échographique représentant l'objet compte tenu d'un signal d'écho reçu en réponse au signal ultrasonore émis en direction de l'objet le long de la deuxième trajectoire,
    dans lequel la deuxième trajectoire est déterminée compte tenu d'une modification concernant la position de l'origine d'un faisceau ultrasonore correspondant à la première trajectoire et/ou la direction d'émission du faisceau ultrasonore correspondant à la première trajectoire.

2. Appareil échographique selon la revendication 1, dans lequel la sonde est conçue en outre pour émettre un faisceau ultrasonore composé du signal ultrasonore en direction de l'objet dans une pluralité de directions et pour recevoir des signaux d'écho respectivement réfléchis par l'objet compte tenu de la pluralité de directions, et dans lequel le processeur est conçu en outre pour détecter, conformément à un critère prédéterminé, au moins une région offrant une faible qualité d'image parmi les régions présentes dans la première image échographique, au moyen des signaux d'écho réfléchis.

3. Appareil échographique selon la revendication 1, dans lequel, lors de la détection de l'au moins une région offrant une faible qualité d'image conformément au critère prédéterminé, le processeur détecte, si une valeur de corrélation relative au premier foyer et acquise au moyen de différentes fonctions d'apodisation est inférieure à une valeur seuil prédéterminée, une région présente dans la première image échographique et correspondant au premier foyer en tant que région offrant une faible qualité d'image.

4. Appareil échographique selon la revendication 1, comprenant en outre un dispositif d'affichage conçu pour afficher la première et/ou la deuxième image échographique, et pour afficher la carte indicatrice de la qualité de la première image échographique compte tenu de l'au moins une région détectée.

**5.** Appareil échographique selon la revendication 1, dans lequel la sonde comprend une matrice de transducteurs consistant en une pluralité de transducteurs, les informations concernant la direction d'émission du faisceau ultrasonore consistant en des informations concernant un angle entre la direction d'émission du faisceau ultrasonore et la matrice de transducteurs.

**6.** Appareil échographique selon la revendication 1, dans lequel le processeur est conçu en outre pour commander le signal ultrasonore afin qu'il soit émis le long d'une troisième trajectoire compte tenu de l'au moins une région et pour générer une deuxième image échographique correspondant à l'objet compte tenu d'un signal d'écho reçu en réponse au signal ultrasonore émis en direction de l'objet le long de la deuxième trajectoire et d'un signal d'écho reçu en réponse au signal ultrasonore émis en direction de l'objet le long de la troisième trajectoire.

**7.** Appareil échographique selon la revendication 1, comprenant en outre une interface utilisateur conçue pour recevoir une entrée utilisateur permettant de régler l'émission du signal ultrasonore le long de la deuxième trajectoire compte tenu de l'au moins une région, et

dans lequel le processeur est conçu en outre pour commander le signal ultrasonore afin qu'il soit émis le long de la deuxième trajectoire compte tenu de l'entrée utilisateur.

**8.** Appareil échographique selon la revendication 1, dans lequel le processeur est conçu en outre pour commander la sonde de manière qu'elle réalise une formation de faisceau au moyen d'un nombre prédéterminé de sous-ouvertures en lesquelles sont divisés une pluralité de transducteurs de la sonde.

**9.** Procédé d'exploitation d'un appareil échographique, le procédé comprenant :

l'émission, par une sonde de l'appareil échographique, d'un signal ultrasonore en direction d'un objet le long d'une première trajectoire, en concentrant le signal ultrasonore en un premier foyer, et la réception d'un signal d'écho réfléchi par l'objet,
la génération d'une première image échographique représentant l'objet et la détection d'au moins une région offrant une faible qualité d'image parmi les régions présentes dans la première image échographique générée, conformément à un critère prédéterminé, et
la génération d'une deuxième image échographique représentant l'objet compte tenu d'un signal d'écho reçu en réponse au signal ultrasonore émis en direction de l'objet le long d'une deuxième trajectoire ;
ladite génération de la deuxième image échographique comprenant :

la génération d'une carte indicatrice de la qualité de la première image échographique compte tenu de l'au moins une région détectée, et
la commande de la sonde compte tenu de la carte de façon que le signal ultrasonore parvienne à l'au moins une région détectée le long de la deuxième trajectoire en concentrant le signal ultrasonore en un deuxième foyer au sein d'une région prédéterminée de l'objet, ladite deuxième trajectoire étant déterminée compte tenu d'un facteur obstruant la première trajectoire ;
ladite deuxième trajectoire étant déterminée compte tenu d'une modification concernant la position de l'origine d'un faisceau ultrasonore correspondant à la première trajectoire et/ou la direction d'émission du faisceau ultrasonore correspondant à la première trajectoire.

**10.** Procédé selon la revendication 9, dans lequel l'émission du signal ultrasonore en direction de l'objet le long de la première trajectoire et la réception du signal d'écho réfléchi par l'objet comprend l'émission d'un faisceau ultrasonore composé du signal ultrasonore en direction de l'objet dans une pluralité de directions et la réception de signaux d'écho respectivement réfléchis par l'objet compte tenu de la pluralité de directions, et

dans lequel la génération de la première image échographique et la détection de l'au moins une région offrant une faible qualité d'image conformément au critère prédéterminé comprend la détection, conformément à un critère prédéterminé, d'au moins une région offrant une faible qualité d'image parmi les régions présentes dans la première image échographique, au moyen des signaux d'écho réfléchis.

**11.** Procédé selon la revendication 9, dans lequel la détection de l'au moins une région offrant une faible qualité d'image conformément au critère prédéterminé comprend la détection, si une valeur de corrélation relative au premier foyer et acquise au moyen de différentes fonctions d'apodisation est inférieure à une valeur seuil prédéterminée, d'une région présente dans la première image échographique et correspondant au premier foyer en tant que région offrant une faible qualité d'image.

**12.** Procédé selon la revendication 9, comprenant en outre l'affichage des deuxièmes images échographiques.

# FIG. 1

EP 3 311 752 B1

# FIG. 2

WIRELESS PROBE 2000

TRANSMITTER 2100

PULSER 2110 → TRANSMISSION DELAYING UNIT 2120 → PULSE GENERATOR 2130

TRANSDUCER 2200

10

RECEIVER 2300

AMPLIFIER 2310 → ADC 2320 → RECEPTION DELAYING UNIT 2330 → SUMMING UNIT 2340

# FIG. 3

300

# FIG. 4

400

# FIG. 5

START

TRANSMIT ULTRASOUND SIGNAL TO OBJECT
ALONG FIRST PATH AND RECEIVE ECHO
SIGNAL REFLECTED FROM OBJECT — S510

GENERATE FIRST ULTRASOUND IMAGE OF OBJECT
BASED ON REFLECTED ECHO SIGNAL AND DETECT
AT LEAST ONE REGION HAVING LOW IMAGE QUALITY — S520
AMONG REGIONS IN FIRST ULTRASOUND IMAGE
ACCORDING TO PREDETERMINED CRITERION

CONTROL ULTRASOUND SIGNAL TO BE TRANSMITTED
ALONG SECOND PATH BASED ON AT LEAST ONE REGION — S530

GENERATE SECOND ULTRASOUND IMAGE OF OBJECT
BASED ON ECHO SIGNAL OF ULTRASOUND SIGNAL — S540
TRANSMITTED TO OBJECT ALONG SECOND PATH

END

## FIG. 6A

610

## FIG. 6B

620

# FIG. 7A

714

712

713

711

710

# FIG. 7B

714

712

713

721

722

711

720

# FIG. 8

FROM S540

DISPLAY AT LEAST ONE OF FIRST AND
SECOND ULTRASOUND IMAGES — S810

DISPLAY MAP INDICATING QUALITY OF FIRST
ULTRASOUND IMAGE BASED ON DETECTED
AT LEAST ONE REGION — S820

END

# FIG. 9

910

920

923

922    921

# FIG. 10

EP 3 311 752 B1

# FIG. 11

FROM S520

RECEIVE USER INPUT FOR TRANSMITTING
ULTRASOUND SIGNAL ALONG SECOND PATH
BASED ON AT LEAST ONE REGION — S1110

CONTROL ULTRASOUND SIGNAL TO BE TRANSMITTED
ALONG SECOND PATH BASED ON USER INPUT — S1120

FROM S540

# FIG. 12

1204

1202

1203

1206

1207

1201

1205

1200

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20100305441 A1
- US 8873830 B
- JP 2007296336 A
- JP 2013244164 A

**Non-patent literature cited in the description**

- **KYUHONG et al.** A fast minimum variance beamforming method using principal component analysis. *IEEE transactions on ultrasonics, ferroelectrics, and frequency control,* 2014, vol. 61.6, 930-945

- **YEN, JESSE T.** Synergistic enhancements of ultrasound image contrast with a combination of phase aberration correction and dual apodization with cross-correlation. *IEEE transactions on ultrasonics, ferroelectrics, and frequency control,* 2012, vol. 59.9, 2089-2101